Europäisches Patentamt

European Patent Office

Office européen des brevets

(11) Publication number: **0 269 876 B1**

# EUROPEAN PATENT SPECIFICATION

(45) Date of publication of patent specification: **18.08.93**    (51) Int. Cl.⁵: **G01N 33/52**, G01N 33/53

(21) Application number: **87115975.2**

(22) Date of filing: **30.10.87**

(54) **Sample focuser for solid-phase analytical device.**

(30) Priority: **24.11.86 US 934043**

(43) Date of publication of application:
**08.06.88 Bulletin  88/23**

(45) Publication of the grant of the patent:
**18.08.93 Bulletin  93/33**

(84) Designated Contracting States:
**BE DE FR IT**

(56) References cited:
**EP-A- 0 182 075**
**EP-A- 0 206 561**
**EP-A- 0 217 403**
**WO-A-50/5451**
**GB-A- 2 090 659**

(73) Proprietor: **ABBOTT LABORATORIES**

**Abbott Park, Illinois 60064(US)**

(72) Inventor: **Varitek, Vincent Andrew**
**33139 North Sunset**
**Wildwood, IL 60030(US)**
Inventor: **Vcelka, John Leonard**
**905 Wilson Court**
**Zion, IL 60099(US)**

(74) Representative: **Modiano, Guido et al**
**c/o Modiano & Associati S.r.l. Via Meravigli,**
**16**
**I-20123 Milano (IT)**

## Description

### Background of the Invention

The present invention is generally directed toward an improved solid-phase assay device for accurately detecting an analyte present in a biological fluid. Generally, the detection of analyte is complicated by the difficulty in obtaining sufficient quantity of sample or by the low concentration of analyte present in such samples. Therefore it is desirable to design a device whereby minimal amounts of sample can be accurately tested and whereby low concentrations of analyte can be detected.

One device which can be employed to identify analyte by performing a binding assay is TESTPACK™ manufactured by Abbott Laboratories, Abbott Park, Ill., and disclosed in EP-A-0 217 403. Generally the device disclosed employs an enzyme immunoassay technique and comprises a plurality of substantially special, solid particles immobilized within a porous matrix of fibers. Generally the particles have substance coated on their surface which is capable of reacting with analyte present in a sample. The sample is passed through the porous matrix where analyte present in the sample binds to the microparticle surface which can be detected colorimetrically.

The device and method as disclosed in the above referenced EP Application is extremely successful in identifying a variety of antigens, antibodies or other substances present in a sample material.

Known from WO-85/05451 teaches an apparatus for immunoassays having a container constricted to provide an integral funnel to direct a sample to an analyte reaction site present on a solid phase assay device.

Also known from EP-A-0,206,561, which belongs to the prior art according to Art. 54(3) EPC, is a device for testing for the presence of an analyte in a liquid which includes a sample focuser as described in the precharacterizing clause of claim 1.

However, further improvements are necessary to identify small concentrations of antigen, antibodies or to analyze small quantities of sample. These problems have been identified and their solutions are the subject matter of the present invention.

### Summary of the invention.

According to one aspect of the invention, the shortcomings of the known sample focusers are overcome by providing a sample focuser as defined in claim 1.

According to another aspect of the invention, the shortcomings of the known sample focusers are overcome by providing a method for determining the presence of an analyte in a liquid sample as defined in claim 11.

The invention is directed toward a sample focuser device suitable for use with a solid-phase assay device. The focuser device is molded or otherwise manufactured with sidewalls and a base for containing a sample material and has passage means for directing a sample to an analyte reactive site present on a solid-phase assay device. The passage means can be configured to expose only a portion of the analyte reactive site, all the analyte reactive site or various areas of the entire reactive matrix.

The focuser can include tabs for easy placement and removal. Further, the focuser can have positioning means such as detents or other means to attach it to the solidphase device whereby the passage means accurately communicate with the analyte reactive site(s).

In another aspect, the focuser can include downwardly depending flanges which engagingly mate an engagement means present on the solid-phase assay device. The combination of the flanges and engagement means present on the solid-phase assay device make the focuser self-positioning. The engagement means can include a circumferential groove present on the sides of the solid-phase assay device.

The focuser device of the present invention provides an effective means for accurately detecting small concentrations of analyte or analyzing small quantities of sample for an analyte. Also, the focuser device allows extreme flexibility in its application to a solid-phase assay device by being easily inserted or removed in order to process a sample.

### Brief Description of the Drawings

Fig. 1 is a side view in partial cross section of a solid-phase analytical device in accordance with the present invention depicting one embodiment of a sample focuser in position.

Fig. 2. is a top plan view of the device of Fig. 1 showing the sample focuser in position.

Fig. 3. is a perspective view of the device of Fig. 1, showing a sample focuser assembly removed from the body of the solid-phase analytical device.

Fig. 4 is a side view in partial cross section of a solid-phase analytical device in accordance with the present invention depicting another embodiment of a sample focuser in position.

Fig. 5 is a top plan view of the device of Fig. 4 showing the sample focuser in position.

Fig. 6 is a perspective view of the device of Fig. 4 showing the sample focuser assembly removed from the body of the solid-phase analytical device.

Detailed Description of the Invention

The present invention is directed toward an improved device for identifying small concentrations of analyte present in a sample and/or identifying the presence of an analyte in a small quantity of sample. In particular the subject sample focuser in adapted for use with the diagnostic device called TESTPACK™ which is commercially available from Abbott Laboratories, Abbott Park, Illinois. However, the focuser is also suitable for use in other analytical or diagnostic devices where concentration of sample material is desirable.

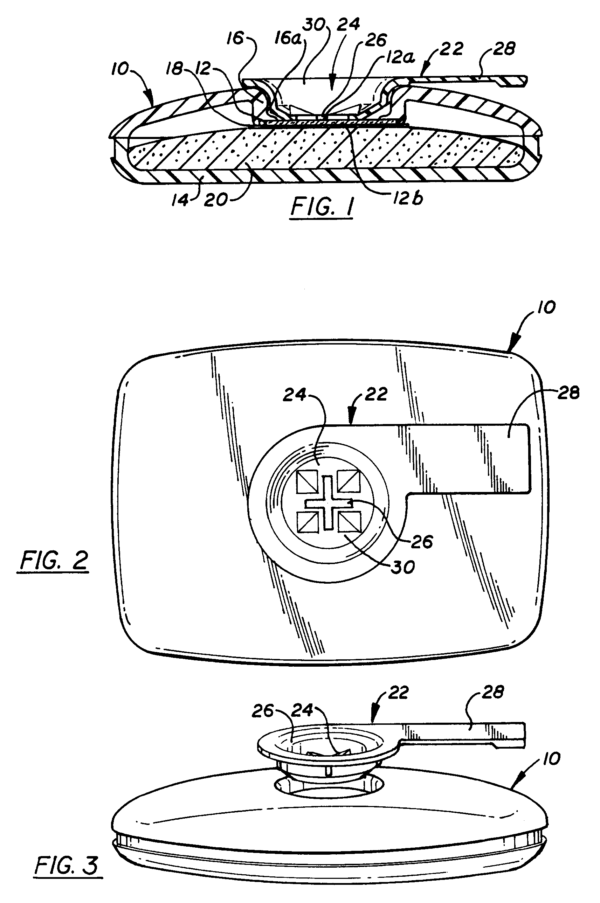

Generally the improved device employs a solid-phase immunoassay technique for performing colormetric or other enzyme immunoassay analysis of biological fluids. The device as depicted in Fig. 1 shows a cross-sectional view of the various components of a typical solid-phase device, in this case TESTPACK.

The device 10 comprises a substantially planar, generally circular, disk-shaped reaction matrix 12. The matrix 12 contains reactive microparticles, or other reaction means necessary for performing an assay, and is disposed within the device 10 such that the various chemical reactions and changes necessary to perform a binding assay can take place therein for visual or instrumental detection.

The matrix 12 has a sample-contacting surface 12a and a surface 12b opposed therefrom. The filter matrix 12 is a "porous" filter matrix meaning that the matrix is composed of a material into which fluids can flow and easily pass through. Appropriate materials can include glass fibers, cellulose, nylon, or other fibrous materials well known in the art. One preferred material is "Whatman GF/D" glass fiber filter paper (Whatman Reeve Angel, Inc., Clifton, New Jersey) which has a nominal thickness of 0.0813 cm (0.032 inch); however, thickness is not a critical factor.

Generally, the device 10 comprises absorbent means 20 disposed in the carrier 14, as shown, for absorbing fluids during use of the assay device. The absorbent means 20 of the device 10 can comprise one or more layers of material and is in physical contact, as shown, with the barrier material 18, when used, or with the reaction matrix 12. This especially advantageous feature enables excess fluid, during the performance of an assay using the device 10, to be easily absorbed, as necessary, after passage of such excess fluid from the reaction matrix 12 during the assay procedure. The absorbent means 20 can be virtually any moisture or fluid-retaining material, e.g., that available from James River, and designated "105 point" or "50 point", or, as is especially preferred, a combination of one or more layers of each of the foregoing.

Barrier means is provided for restricting fluid flow in the solid phase analytical devices. This aspect is particularly advantageous when used in solid phase analytical devices having a permeable reaction surface or matrix, or filter layer, and an absorbant layer for absorbing fluids used in the device to permit the flow of fluids from the reaction surface to the absorbant means or layer while preventing the back flow of fluids from the absorbant layer to the reaction matrix.

As shown in Fig. 1, the barrier means comprises a layer of barrier material 18 extending under the matrix 12 and within the carrier 14. The barrier material 18 is in contact with the surface 12b of the matrix 12, and functions, when the device is in use, to restrict fluid passing through the matrix 12, to and through the surface 12b, and into the layer 18, from re-contacting the surface 12b. Layer 18 is employed as a fluid restrictive layer and to help prevent or eliminate "background" interference in the matrix 12. However, this feature is not essential or critical to the basic functions or concepts of the matrix 12, and can be omitted from the device if desired. If omitted, the device generally will perform satisfactorily in an assay, but possibly with less sensitivity (diminished detectable response).

The layer 18 can comprise any suitable material capable of restrictive, substantially "one-way" flow of fluid or moisture. Examples of especially suitable materials for this purpose are polyethylene weave materials manufactured and sold by Ethyl Visqueen Corp., Baton Rouge, Louisiana under the designations "X-6057" (1.0 mil) and "X-6108" (1.25 mil) as well as those materials described in U.S. Patents 3,939,135 and 4,342,314. Another effective material is Lydair Grade 254 from Lydall.

The device 10 additionally includes a carrier 14 within which the matrix 12 is disposed. The carrier 14 can be made of any suitable material such as plastic, metal or other rigid or semi-rigid substance.

EP 0 269 876 B1

Especially preferred as a material for the carrier 14 is a plastic commercially known as "ABS", and available from the Monsanto Company, St. Louis, Missouri. In the preferred embodiment shown, the carrier 14 completely surrounds the matrix 12 and functions as a support and holder therefore. In order to accomplish his function, the carrier 14 has a generally circular flange 16 for supporting and holding tightly the matrix 12. A fluid chamber is generally defined by sidewalls formed by an outer wall surface 16a of the flange 16 and a base wall formed by the sample contacting surface 12a of the matrix 12.

The fluid chamber also operates as the receiving means for the subject sample focuser. Generally the reactive portion of the reaction matrix 12 is a printed or impregnated area of limited size. For example, the surface area of the reaction matrix 12 or sample-contacting surface 12a may be 180mm$^2$ where only 15 mm$^2$ is analyte specific, i.e., position where microparticles or other material reactive with the analyte is placed on the fiber matrix. Thus, only that portion of the sample which comes in contact with the activated area can be detected. To increase the opportunity for analyte capture by the reaction matrix, the subject sample focuser is employed.

The sample focuser 22 effectively focuses the sample over the reactive areas of reaction matrix 12, thereby increasing the sensitivity of the assay. This is accomplished by manufacturing a cup or other vessel means 24 having passage means 26 corresponding to the size and orientation of the reactive areas present on a reaction matrix 12. Generally the focuser is molded of a plastic material which has low permeability, is nonreactive with the sample and does not hinder drainage of the sample to be tested. One preferred plastic is polypropylene. The sample focuser is adapted to engagingly fit the sample receiving area of a diagnostic device or with reference to Fig. 1 the fluid chamber defined by the outer wall surface 16a of the flange 16.

Figures 1, 2 and 3 depict one embodiment of a sample focuser 22 which engagingly fits into the fluid chamber as defined by flange 16 and has a tab means 28 to assist in removal.

The cup or vessel means 24 is constructed to hold an appropriate amount of sample for purposes of performing an assay. Generally, the cup or vessel means 24 holds from about .5 milliliters to about 1 milliliter of sample. The interior sides 30 of the cup or vessel means 24 are downwardly slanted to assure complete drainage of the sample. Optionally the cup or vessel means 24 can be treated to be hydrophobic whereby any sample containment is avoided.

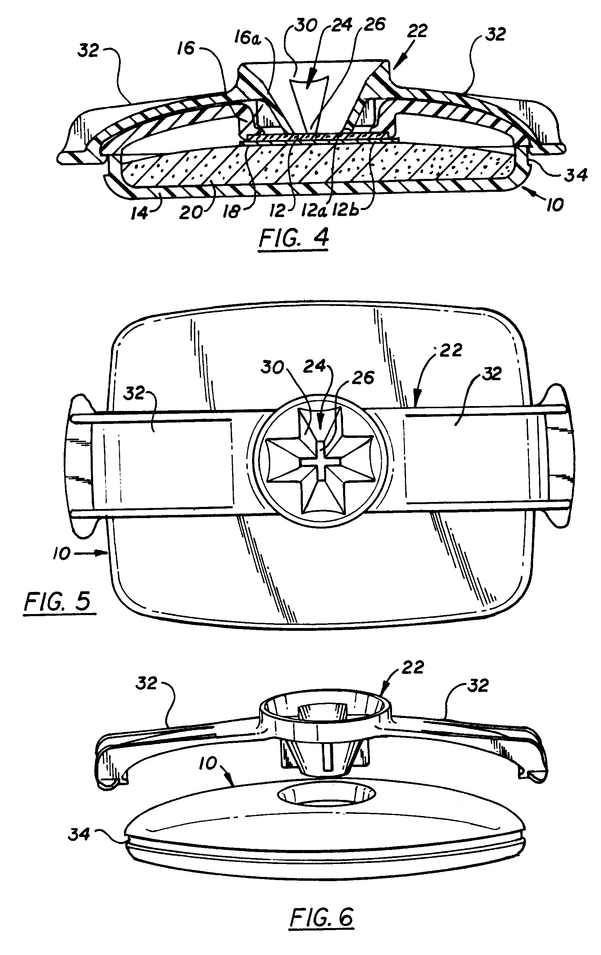

Figures 4, 5 and 6 depict another embodiment of a sample focuser 22 which engagingly fits into the fluid chamber and additional has downwardly depending flanges 32 which engage a circumferential groove 34 present on the device 10 on opposite sides. The design of the latter focuser allows it to be self-positioning, thereby reducing any risk of improper placement and, insuring the passage means 26 are perfectly coincident with the analyte specific area of the reaction matrix 12.

Further, any of the sample focusers described above can additionally include a filter means. The filter means can be formed of any fibrous or porous material suitable for filtering the sample prior to contacting the reaction matrix 12. Such filtering means can include porous of fibrous material such as glass or cellulose filter membrane; especially preferred are "Lydair™ Grade 254" from Lydall, Manchester, Connecticut, and "GF/F" or "GF/D" (glass fiber, filter paper) from whatman Reeve Angel, Inc., Clifton, New Jersey, either singly or in combination. Generally, the filter means is attached to the bottom surface of the focuser whereby sample first passes through the filter means prior to contacting the surface of a reaction matrix.

Figures 1 and 2 show one embodiment of the sample focuser 22 in place over the reaction matrix 12 having a reaction area configured as a cross. The sample focuser 22 further comprises a tab means 28 for displacing the sample focuser 22. The passage means 26 of the sample focuser 22 can also be configured to other types of reaction areas such as dots, circles, dashes, etc.

In addition, the sample focuser 22 can provide means for properly positioning the passage means over the analyte reactive areas of the reaction matrix 12. This is especially true for the focuser shown in Figures 1, 2 and 3. Such positioning means can comprise a detent or other molded feature of the sample focuser which correspondingly engages the analytical device. In the case of the focuser shown in Figs. 4, 5 and 6 the focuser is self-positioning by means of flanges 32 which engagingly secure the focuser to the device 10.

Even though device 10, as described above, can produce a visually-readable response an instrumental determination can also be made of a detectable response therefrom, e.g., corresponding to the reflectance of visible light, or intensity of fluorescence or the like, produced by the matrix 12 as a result of the chemical and biological reactions and changes which occur therein when an assay is performed. Accordingly, the detectable response from the device 10 can be measured by a reflectometer.

A generalized example of how a direct solid-phase enzyme immunoassay is performed with the instant device is as follows:

4

Step a) Pour or otherwise apply a test sample containing the analyte to be determined through the sample focuser positioned over a reaction matrix having an analyte reactive substance present thereon; (the sample focuser can either be removed or left in place for the remaining steps)

Step b) Application of an enzyme-conjugated antibody or antigen to the reaction matrix of Step a);

Step c) Washing, to remove unbound material; and

Step d) Application of an indicator substance which, in the presence of the enzyme portion of the conjugate of Step b), produces a detectable color or other response in the reaction matrix.

The device is not limited to direct assays but can also be advantageously employed for indirect solid-phase enzyme immunoassays as follows:

Step a) pour or otherwise apply a test sample containing analyte to be determined through the sample focuser positioned over a reaction matrix;

Step b) apply analyte specific antibody or antigen through the sample focuser;

Step c) apply enzyme conjugated antibody or antigen through the sample focuser;

Step d) remove sample focuser;

Step e) wash to remove unbound material;

Step f) add indicator, which in the presence of the enzyme portion of the conjugate of step (c) produces a detectable color or other response in the reaction matrix.

Step g) wash and read response.

Generally the solid-phase analytical device provides "on board" control areas to simultaneously display detectable responses corresponding to a positive control (which will display a detectable response indicative of a valid assay result, regardless of the presence or absence of an analyte of interest in a test sample), and a negative control (which will display a detectable response change only if the assay results are invalid). In this aspect of the invention, the same volume of a test sample and assay reagents are simultaneously placed in contact with the procedural controls and test areas, thereby avoiding the necessity of separate control tests as generally practiced in the art. The sample focuser is easily adapted to have passages communicating with each of the test areas to take advantage of these controls and focus sample to the reaction sensitive areas.

On-board negative and positive control areas can be provided on the reaction surface or matrix 12 of the analytical device 10 with the focuser having a passage opening to the respective area. An analyte binding area indicates a positive test result to the user and can be configured to provide a readily recognizable result to the user. More information regarding on-board control areas can be found in U.S. Serial No. 831,013 filed February 18, 1986, herein incorporated by reference.

It has been discovered that when the sample focuser is employed with a solid-phase analytical device, improved accuracy and sensitivity is developed. The following examples demonstrate the improvements provided by the focuser.

Example 1

Two groups of fluid biological samples were prepared with varying concentrations of analyte. The samples were tested on a standard TESTPACK device having a circular reactive site 3 mm in diameter on a surface area of about 176 mm$^2$. The samples were applied with and without a focuser and the difference in sensitivity measured. Measurements were conducted by measuring the reflectance of the reactive sites when exposed to equal amounts of sample (400 $\mu$l). The lower the reflectance the greater the sensitivity, i.e., darker the reactive site became on exposure. The results were as follows:

| Organism[1]/ml (Log 10) | Spot % Reflectance | |
|---|---|---|
| | Unfocused | Focused |
| 5.69 | 40.2 | 33.5 |
| 4.69 | 59.7 | 55.4 |
| 4.00 | 63.3 | 61.0 |
| Negative[2] | 66.3 | 66.1 |

| Organism[3]/ml (Log 10) | Spot % Reflectance | |
|---|---|---|
| | Unfocused | Focused |
| 6.69 | 14.5 | 6.5 |
| 4.69 | 50.2 | 40.4 |
| 3.69 | 61.0 | 58.1 |
| Negative[2] | 64.3 | 63.5 |

[1] Organism was <u>Chlamydia trachomatis</u>.
[2] Negative was a sample containing no organism.
[3] Organism was <u>Neisseria gonorrhoeae</u>.

The data indicates that the focuser effectively increased the sensitivity of the assay device by causing a darker (lower reflectance) color change to be observed at the reaction site.

Example 2

One hundred and twenty urogenital samples were collected from patients and submitted for diagnosis of gonorrhea by standard culture techniques. The samples were tested in a TESTPACK™ solid-phase assay device specific for gonorrhea. Thirty of the cultures were positive and ninety of the cultures were negative. Each extracted sample was evenly divided to permit testing on both an unfocused assay device and focused assay device for direct comparison. The number of the 30 samples the device correctly identified as positive for organism is indicative of sensitivity and the number of the 90 samples the device correctly identified as negative is indicative of specificity. The tests were run as in Example I with any visual change in color of the reactive site being designated as positive and no visual color change of the reactive site being designated as negative. The results were as follows:

## 30 Positive Samples

| Device | Reading | | Sensitivity |
|---|---|---|---|
| | # Positive | # Negative | |
| Focused | 27 | 3 | 90% |
| Unfocused | 24 | 6 | 80% |

## 90 Negative Samples

| Device | Reading | | Specificity |
|---|---|---|---|
| | # Positive | # Negative | |
| Focused | 2 | 88 | 97.8% |
| Unfocused | 2 | 88 | 97.8% |

The data indicates that the focused device had a 10% improvement in sensitivity over the unfocused device. Specificity was not improved but this is not unexpected because the negative results are more a result of the binding properties of the reactive material than exposure. However, the test does indicate that

6

specificity is not reduced when the focuser increases the exposure of the reactive material to the sample.

Overall, it is evident that the focuser improves the function of a solid-phase assay device.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

**Claims**

1. A sample focuser suitable for use with a solid-phase assay device (10, 12, 14, 16, 18, 20) having an analyte reaction site in a reaction matrix (12) housed in a carrier (14), said focuser (22) including side walls (30) and a base (24) for containing a fluid sample material, and passage means (26) for directing said fluid sample to the analyte reaction site, characterized in that it further comprises at least one tab portion (28, 32) for removably displacing or positioning said focuser (22) with respect to said carrier (14).

2. A sample focuser according to claim 1, characterized in that said side walls (30) include a molded portion or detent for positioning said focuser (22) on the carrier (14) of said solid-phase assay device (10, 12, 14, 16, 18, 20).

3. A sample focuser according to claim 1 or 2, characterized in that said at least one tab portion (32) comprises downwardly depending flanges (32) to engagingly mate an engagement means (34) present on the carrier (14).

4. A sample focuser according to claim 3, characterized in that said flanges (32) have a tab means for disengaging said flange (32) from said engagement means (34).

5. A sample focuser according to claim 3 or 4, characterized in that said engagement means (34) comprises a circumferential groove (34) present on a side of said carrier (14).

6. A sample focuser according to claim 3 or 4, characterized in that said engagement means comprise detents.

7. A sample focuser according to claim 1, characterized in that said passage means (26) defines a configuration resembling a "plus" sign.

8. A sample focuser according to claim 1, 6 or 7, characterized in that said passage means (26) correspond to the size and orientation of said onboard control areas present on said reaction site of said reaction matrix (12).

9. A sample focuser according to claim 1, 6, 7 or 8, characterized in that said passage means (26) is configured as a cross.

10. A sample focuser according to claim 1, 2, 3, 4, 5, 6, 7, 8, or 9, comprising interior walls (30) defining vessel means (24), characterized in that said interior walls (30) are treated to be hydrophobic whereby sample containment is avoided.

11. An assay method employing a sample focuser as defined in Claim 1, for determining the presence of analyte in a liquid sample, said method employing: i) a solid-phase assay device having a reaction site on a reaction matrix; ii) said sample focuser having side walls defining a passage means through said focuser with an opening having an area less than the area of the reaction matrix, and tab means for positioning or displacing said focuser with respect to said assay device; and iii) a reagent system including a labelling component for signalling the presence of analyte in the reaction matrix, said method comprising:

    a) applying to the reaction site via the passage means of the sample focuser, at least one of said fluid sample and said labelling component of the reagent system, while the focuser is attached to the assay device with said opening substantially aligned with the reaction site;

    b) removing the sample focuser; and

c) observing the reaction site for detectable signal.

**12.** The method according to claim 11 wherein both sample and labelling component are added to the reaction matrix through the passage means of said focuser.

**13.** The method according to claim 11 wherein sample only is added via the passage means of said sample focuser, and said sample focuser is removed prior to the addition of further reagents.

**14.** The method according to claim 11 wherein said labelling component comprises and enzyme conjugate, and further comprising the step of adding an indicator substance to the reaction matrix.

**15.** The method according to claim 11 wherein the passage means is configured in the shape of a cross.

**Patentansprüche**

**1.** Probenausrichtungsvorrichtung geeignet zur Verwendung in einem Festphasenanalysator (10, 12, 14, 16, 18, 20) der eine Analysatreaktionsstelle in einer Reaktionsmatrix (12) enthält, die in einem Behälter (14) untergebracht ist, wobei die Ausrichtungsvorrichtung (22) aus seitlichen Wänden (30) und einer Grundplatte (24) zur Aufnahme von flüssigem Probenmaterial besteht, und Durchlaßeinrichtungen (26), um die flüssige Probe zur Analysatorreaktionsstelle zu leiten, dadurch **gekennzeichnet,** daß diese weiterhin zumindest einen Vorsprung (28, 32) hat, um die Ausrichtungsvorrichtung (22) relativ zum Behälter (14) entnehmbar zu verschieben oder zu positionieren.

**2.** Probenausrichtungsvorrichtung gemäß Anspruch 1, dadurch **gekennzeichnet,** daß die seitlichen Wände (30) einen Preßteil oder einen Anschlag zur Positionierung der Probenausrichtungsvorrichtung (22) auf den Behälter (14) des Festphasenanalysators (10, 12, 14, 16, 18, 20) enthalten.

**3.** Probenausrichtungsvorrichtung gemäß Anspruch 1 oder 2, dadurch **gekennzeichnet,** daß der zumindest eine Vorsprung (32) nach unten gerichtete Flanschen (32) enthält, um eingreifbar in eine Eingriffseinrichtung (34) auf dem Behälter (14) einzurasten.

**4.** Probenausrichtungsvorrichtung gemäß Anspruch 3, dadurch **gekennzeichnet,** daß die Flanschen (32) eine Klappeneinrichtung zur Lösung des Flansches (32) von der Eingriffseinrichtung (34) besitzt.

**5.** Probenausrichtungsvorrichtung gemäß Anspruch 3 oder 4, dadurch **gekennzeichnet,** daß die Eingriffseinrichtung (34) eine an der Seite des Behälters (14) umlaufende Kerbe (34) hat.

**6.** Probenausrichtungsvorrichtung gemäß Anspruch 3 oder 4, dadurch **gekennzeichnet,** daß die Eingriffseinrichtung eine Arretierung hat.

**7.** Probenausrichtungsvorrichtung gemäß Anspruch 1, dadurch **gekennzeichnet,** daß die Durchlaßeinrichtung (26) eine Anordnung definiert, die die Form eines "plus"-Zeichens bildet.

**8.** Probenausrichtungsvorrichtung gemäß Anspruch 1, 6 oder 7, dadurch **gekennzeichnet,** daß die Durchlaßeinrichtungen (26) in Größe und Ausrichtung denen auf der Reaktionsstelle der Reaktionsmatrix (12) befindlichen Kontrollabschnitten entsprechen.

**9.** Probenausrichtungsvorrichtung gemäß Anspruch 1, 6, 7 oder 8, dadurch **gekennzeichnet,** daß die Durchlaßeinrichtung (26) kreuzförmig angeordnet ist.

**10.** Probenausrichtungsvorrichtung gemäß Anspruch 1, 2, 3, 4, 5, 6, 7, 8 oder 9, bestehend aus inneren Wänden (30), die ein Gefäßdefinierendesmittel (24) bilden, dadurch **gekennzeichnet,** daß die inneren Wände (30) hydrophob sind, wodurch das Zurückbleiben einer Probe vermieden wird.

**11.** Analyseverfahren, das eine Probenausrichtungsvorrichtung gemäß Anspruch 1 verwendet, um die Anwesenheit eines Analysats in einer flüssigen Probe zu messen, wobei das Verfahren verwendet: i) einen Festphasenanalysator mit einer Reaktionsmatrix; ii) wobei die Probenausrichtungsvorrichtung seitliche Wände hat, die eine Durchlaßeinrichtung durch die Probenausrichtungsvorrichtung definieren mit einer Öffnung, die einen Querschnitt hat, der kleiner ist als der Querschnitt der Reaktionsmatrix und einem Mittel, das einen Vorsprung zur relativen Positionierung und Verschiebung der Probenausrichtungsvorrichtung am Analysator bildet; und iii) ein Reagenzsystem mit einer Markierungskomponente, um die Anwesenheit eines Analysats in der Reaktionsmatrix anzuzeigen, wobei das Verfahren umfaßt:

a) Einbringung mindestens einer flüssigen Probe und der Markierungskomponente des Reagenzsystems in die Reaktionsstelle über der Durchlaßeinrichtung der Probenausrichtungsvorrichtung, wobei die Probenausrichtungsvorrichtung an den Analysator gekoppelt ist und die Öffnung im wesentlichen zur Reaktionsstelle ausgerichtet ist;

b) Entfernung der Probenausrichtungsvorrichtung; und

c) Beobachtung der Reaktionsstelle auf ein erfaßbares Signal hin.

12. Verfahren nach Anspruch 11, dadurch **gekennzeichnet,** daß sowohl Probe als auch Markierungskomponente der Reaktionsmatrix über die Durchlaßeinrichtung der Probenausrichtungsvorrichtung zugefügt werden.

13. Verfahren nach Anspruch 11, dadurch **gekennzeichnet,** daß nur die Probe über die Durchlaßeinrichtung der Probenausrichtungsvorrichtung hinzugefügt wird und die Probenausrichtungsvorrichtung vor der Zufügung weiterer Reagenzien entfernt wird.

14. Verfahren nach Anspruch 11, dadurch **gekennzeichnet,** daß die Markierungskomponente eine Enzymverbindung enthält, und weiterhin den Schritt der Zumischung einer Indikatorsubstanz zur Reaktionsmatrix enthält.

15. Verfahren nach Anspruch 11, dadurch **gekennzeichnet,** daß die Durchlaßeinrichtung in Form eines Kreuzes gestaltet ist.

## Revendications

1. Dispositif de focalisation d'échantillon approprié à une utilisation avec un dispositif de dosage en phase solide (10, 12, 14, 16, 18, 20) ayant un site de réaction d'un analyte dans une matrice réactionnelle (12) contenue dans un support (14), ledit dispositif de focalisation (22) comportant des parois latérales (30) et une base (24), pour contenir un matériau échantillon fluide, et un moyen de passage (26), pour diriger ledit échantillon fluide vers le site de réaction de l'analyte, caractérisé en ce qu'il comprend en outre au moins une partie servant de patte (28, 32) pour déplacer ou positionner ledit dispositif de focalisation amovible (22) par rapport audit support (14).

2. Dispositif de focalisation d'échantillon selon la revendication 1, caractérisé en ce que lesdites parois latérales (30) comportent une portion moulée ou un cliquet de blocage pour positionner ledit dispositif de focalisation (22) sur le support (14) dudit dispositif de dosage en phase solide (10, 12, 14, 16, 18, 20).

3. Dispositif de focalisation d'échantillon selon la revendication 1 ou 2, caractérisé en ce que ladite au moins une partie servant de patte (32) comprend des brides de sécurité dirigées vers le bas (32) qui s'ajustent par enclenchement dans un moyen d'accrochage (34) présent sur le support (14).

4. Dispositif de focalisation d'échantillon selon la revendication 3, caractérisé en ce que lesdites brides (32) comportent une moyen constituant une patte pour désengager ladite bride (32) dudit moyen d'accrochage (34).

5. Dispositif de focalisation d'échantillon selon la revendication 3 ou 4, caractérisé en ce que ledit moyen d'accrochage (34) comprend une rainure circonférentielle (34) présente sur un côté dudit support (14).

6. Dispositif de focalisation d'échantillon selon la revendication 3 ou 4, caractérisé en ce que ledit moyen d'accrochage comprend des cliquets de blocage.

7. Dispositif de focalisation d'échantillon selon la revendication 1, caractérisé en ce que ledit moyen de passage (26) définit une configuration ressemblant à un signe "plus".

**8.** Dispositif de focalisation d'échantillon selon la revendication 1, 6 ou 7, caractérisé en ce que ledit moyen de passage (26) correspond à la dimension et à l'orientation desdites zones témoin intégrées présentes sur ledit site de réaction de ladite matrice réactionnelle (12).

**9.** Dispositif de focalisation d'échantillon selon la revendication 1, 6, 7 ou 8, caractérisé en ce que ledit moyen de passage (26) est configuré sous la forme d'une croix.

**10.** Dispositif de focalisation d'échantillon selon la revendication 1, 2, 3, 4, 5, 6, 7, 8 ou 9, comprenant des parois intérieures (30), définissant un moyen constituant un réservoir (24) caractérisé en ce que lesdites parois intérieures (30) sont traitées pour être hydrophobes, cela évitant la rétention d'échantillon.

**11.** Méthode de dosage utilisant un dispositif de focalisation d'échantillon tel que défini dans la revendication 1, pour déterminer la présence d'un analyte dans un échantillon liquide, ladite méthode utilisant :
   i) un dispositif de dosage en phase solide ayant un site de réaction sur une matrice réactionnelle ;
   ii) ledit dispositif de focalisation d'échantillon ayant des parois latérales définissant un moyen de passage au travers dudit dispositif de focalisation avec un orifice ayant une superficie inférieure à la superficie de la matrice réactionnelle et un moyen constituant une patte pour positionner ou déplacer ledit dispositif de focalisation par rapport audit dispositif de dosage et iii) un système de réactifs incluant un composant de marquage pour signaler la présence de l'analyte dans la matrice réactionnelle, la dite méthode comprenant :
   a) l'application au site de réaction par l'intermédiaire du moyen de passage du dispositif de focalisation d'échantillon, d'au moins un dudit échantillon fluide et dudit composant de marquage du système de réactifs, alors que le dispositif de focalisation est fixé sur le dispositif de dosage avec ledit orifice pratiquement aligné avec le site de réaction.
   b) l'enlèvement du dispositif de focalisation d'échantillon ; et
   c) l'observation du site de réaction pour rechercher le signal détectable.

**12.** Méthode selon la revendication 11 dans laquelle l'échantillon et le composant de marquage sont ajoutés tous deux à la matrice réactionnelle par le moyen de passage dudit dispositif de focalisation.

**13.** Méthode selon la revendication 11 dans laquelle l'échantillon seul est ajouté par l'intermédiaire du moyen de passage dudit dispositif de focalisation d'échantillon, et ledit dispositif de focalisation d'échantillon est enlevé avant l'addition des autres réactifs.

**14.** Méthode selon la revendication 11 dans laquelle le composant de marquage comprend un conjugué enzymatique et comprenant en outre l'étape d'additionner une substance indicatrice à la matrice réactionnelle.

**15.** Méthode selon la revendication 11 dans laquelle le moyen de passage est configuré de façon à avoir la forme d'une croix.

FIG. 1

FIG. 2

FIG. 3

FIG. 4

FIG. 5

FIG. 6